(19) Europäisches Patentamt
European Patent Office
Office européen des brevets

(11) Veröffentlichungsnummer: **0 492 496 A2**

# (12) EUROPÄISCHE PATENTANMELDUNG

(21) Anmeldenummer: **91121932.7**

(22) Anmeldetag: **20.12.91**

(51) Int. Cl.5: **C07C 59/135**, C07C 51/487, C07C 51/41

(30) Priorität: **24.12.90 DE 4041718**

(43) Veröffentlichungstag der Anmeldung: **01.07.92 Patentblatt 92/27**

(84) Benannte Vertragsstaaten: **DE FR GB IT NL**

(71) Anmelder: **HOECHST AKTIENGESELLSCHAFT**
**Postfach 80 03 20**
**W-6230 Frankfurt am Main 80(DE)**

(72) Erfinder: **Gries, Thomas, Dr.**
**Johannesallee 18**
**W-6230 Frankfurt am Main 80(DE)**
Erfinder: **Ginzel, Klaus-Dieter, Dr.**
**Homburger Strasse 11**
**W-6238 Hofheim am Taunus(DE)**
Erfinder: **Ebmeyer, Frank Dr.**
**Palleskestrasse 29**
**W-6230 Frankfurt am Main(DE)**

## (54) Verfahren zur Rückgewinnung von Perfluorethercarbonsäuren.

(57) Die Erfindung betrifft ein Verfahren zur Rückgewinnung von Perfluorethercarbonsäuren aus dem Rohprodukt der elektrochemischen Umsetzung dieser Säuren oder eines ihrer löslichen Salze zu perfluorierten Ethern, bei dem man die in dem Rohprodukt enthaltenen Perfluorethercarbonsäuren und deren Ester mit Hilfe eines festen basischen Alkalimetallsalzes oder dessen Lösung abtrennt und durch Reaktion der dabei entstandenen Salze mit einer starken Säure in die entsprechenden Perfluorethercarbonsäuren überführt.

EP 0 492 496 A2

Die Erfindung betrifft ein Verfahren zur Rückgewinnung von Perfluorethercarbonsäuren aus Verunreinigungen, die bei der elektrochemischen Umsetzung von Perfluorethercarbonsäuren zu perfluorierten Ethern im Rohprodukt anfallen.

Die zurückgewonnenen Perfluorethercarbonsäuren können erneut zur Synthese perfluorierter Ether eingesetzt werden. Dadurch wird die Ausbeute der Synthese erhöht und die Abfallmenge vermindert.

Die Herstellung perfluorierter Ether gelingt in guten Ausbeuten durch Kolbe-Elektrolyse perfluorierter Ethercarbonsäuren oder deren löslicher Salze (JP Sho- 58 103 334, DE-OS 3 828 848):

$2\ R_f\text{-}COOH \rightarrow R_f\text{-}R_f + 2CO_2 + H_2$

worin
$R_f = C_aF_{2a+1}O\text{-}(C_3F_6O)_m\text{-}CF(CF_3)\text{-}$
mit a = 1,2 oder 3
und m = 0,1,2 oder 3 ist.

Die bei dieser Elektrolyse eingesetzten Perfluorethercarbonsäuren werden hergestellt durch Oligomerisierung von Hexafluorpropylenepoxid (HFPO) zu Carbonsäurefluoriden und deren Hydrolyse zu Perfluorethercarbonsäuren:

$$nHFPO \longrightarrow C_3F_7O\text{-}(CF(CF_3)\text{-}CF_2\text{-}O)_{n-2}CF(CF_3)\text{-}COF \longrightarrow$$

$$\xrightarrow{H_2O} C_3F_7O\text{-}(CF(CF_3)\text{-}CF_2\text{-}O)_{n-2}CF(CF_3)\text{-}COOH$$

Die dabei gebildeten Perfluorethercarbonsäuren werden anschließend destillativ getrennt. Aber auch nach der Destillation kann eine solche Perfluorethercarbonsäure noch mit dem nächsthöheren oder nächstniederen Homologen oder beiden verunreinigt sein, i. allg. ist deren Anteil aber weniger als 5 Gew.%. Elektrolysiert man ein Gemisch aus zwei Carbonsäuren $R_f$-COOH und $R_f'$-COOH bzw. deren Salzen, so erhält man ein Gemisch der Perfluorether $R_f$-$R_f$, $R_f'$-$R_f'$ und $R_f$-$R_f'$. Analoges gilt, wenn drei Carbonsäuren im Gemisch vorliegen.

Die durch Elektrolyse der Perfluorethercarbonsäuren hergestellten Perfluorether enthalten als Verunreinigungen unter anderem unumgesetzte Perfluoroethercarbonsäuren sowie deren Ester, die durch Reaktion der Carbonsäuren mit dem für die Elektrolyse verwendeten Lösungsmittel (einem Alkohol oder einer Alkohol-Wasser-Mischung) entstehen. Geeignete Alkohole sind dabei z.B. Methanol, Ethanol, Propanol, Butanol, Pentanol, Glykol, Diethylenglykol sowie Triethylenglykol, insbesondere Methanol und Ethanol.

Ein Verfahren zur vollständigen Abtrennung der Verunreinigungen durch thermische Zersetzung zu nicht mehr verwertbaren Stoffen ist in der DE-OS 3 902 803 beschrieben. Da die Verunreinigungen jedoch zu einem hohen Anteil aus wertvollem perfluorierten Material bestehen, ist es wünschenswert, dieses soweit wie möglich zurückzugewinnen und erneut zur Synthese perfluorierter Ether einzusetzen.

Gegenstand der Erfindung ist demgemäß ein Verfahren zur Rückgewinnung von Perfluorethercarbonsäuren der allgemeinen Formel I

(I) $R_f$-COOH, worin

$R_f = C_aF_{2a+1}O\text{-}(C_3F_6O)_m\text{-}CF(CF_3)\text{-}$
mit a = 1,2 oder 3
und m = 0,1,2 oder 3 ist
aus dem Rohprodukt der elektrochemischen Herstellung perfluorierter Ether, dadurch gekennzeichnet, daß man die in dem Rohprodukt enthaltene Perfluorethercarbonsäure der allgemeinen Formel I und deren Ester der allgemeinen Formel II

(II) $R_f$-COOR, worin

$R_f$ die unter (I) angegebene Bedeutung hat und
$R = C_cH_{2c+1}$ mit c = 1,2,3,4 oder 5

oder
$R = -C_dH_{2d+1}O_e$ mit d = 2,3,4,5 oder 6
und e = 1,2 oder 3 ist
mit Hilfe eines festen basischen Alkalimetallsalzes oder dessen Lösung abtrennt und durch Reaktion der dabei entstandenen Salze mit einer starken Säure in die entsprechende Perfluorethercarbonsäure der allgemeinen Formel I überführt.

Aus dem Rohprodukt der elektrochemischen Umsetzung eines Gemisches von zwei Perfluorethercarbonsäuren lassen sich diese analog zurückgewinnen. Ein weiterer Gegenstand der Erfindung ist daher ein Verfahren zur Rückgewinnung der beiden Perfluoroethercarbonsäuren der allgemeinen Formeln I und I'

(I) $R_f$-COOH, worin

$R_f = C_aF_{2a+1}O-(C_3F_6O)_m-CF(CF_3)-$
mit a = 1,2 oder 3 und m = 0,1,2 oder 3 ist

(I') $R_f'$-COOH, worin

$R_f' = C_bF_{2b+1}O-(C_3F_6O)_n-CF(CF_3)$
mit b = 1,2 oder 3 und n = 0,1,2 oder 3 ist
aus dem Rohprodukt der elektrochemischen Umsetzung dieser Säuren oder ihrer löslichen Salze zu perfluorierten Ethern, dadurch gekennzeichnet, daß man die in dem Rohprodukt enthaltenen Perfluorethercarbonsäuren der allgemeinen Formeln I und I' und deren Ester der allgemeinen Formeln II und II'

(II) $R_f$-COOR

(II') $R_f'$-COOR

worin $R_f$ und $R_f'$ die unter (I) und (I') angegebene
Bedeutung haben
und $R = C_cH_{2c+1}$ mit c = 1,2,3,4 oder 5
oder $R = -C_dH_{2d+1}O_e$ mit d = 2,3,4,5 oder 6
und e = 1,2 oder 3 ist
mit Hilfe eines festen basischen Alkalimetallsalzes oder dessen Lösung abtrennt und durch Reaktion der dabei entstandenen Salze mit einer starken Säure in die entsprechenden Perfluorethercarbonsäuren der allgemeinen Formeln I und I' überführt.

Falls nur eine Perfluorethercarbonsäure elektrolysiert wurde, läßt sich diese analog zurückgewinnen. Ein weiterer Gegenstand der Erfindung ist demgemäß ein Verfahren zur Rückgewinnung einer Perfluorethercarbonsäure der allgemeinen Formel I

(I) $R_f$-COOH, worin

$R_f = C_aF_{2a+1}O-(C_3F_6O)_m-CF(CF_3)-$
mit a = 1,2 oder 3
und m = 0,1,2 oder 3 ist
aus dem Rohprodukt der elektrochemischen Umsetzung dieser Säure oder eines ihrer löslichen Salze zu einem perfluorierten Ether, dadurch gekennzeichnet, daß man die in dem Rohprodukt enthaltene Perfluorethercarbonsäure der allgemeinen Formel I und deren Ester der allgemeinen Formel II

(II) $R_f$-COOR, worin

$R_f$ die unter (I) angegebene Bedeutung hat und
$R = C_cH_{2c+1}$ mit c = 1,2,3,4, oder 5
oder
$R = C_dH_{2d+1}O_e$ mit d = 2,3,4,5 oder 6
und e = 1,2 oder 3 ist
mit Hilfe eines festen basischen Alkalimetallsalzes oder desen Lösung abtrennt und durch Reaktion der dabei entstandenen Salze mit einer starken Säure in die entsprechende Perfluorethercarbonsäure der allgemeinen Formel I überführt.

Die Abtrennung der Perfluorethercarbonsäuren und der Ester kann durch Flüssig-Flüssig-Extraktion mit basischen Alkalimetallsalzlösungen oder mit Hilfe fester basischer Alkalimetallsalze, z.B. durch Perkolation des Rohprodukts über ein Alkalimetallhydroxid- oder -carbonat-Festbett erfolgen. Vorzugsweise setzt man dabei basische Na- oder K-Salze bzw. deren Lösungen ein, insbesondere die Hydroxide oder Carbonate von Na oder K. Im allgemeinen verwendet man die Alkalimetallsalze in 1 bis 5 fachem Überschuß. Die Konzentration der Alkalimetallsalzlösungen beträgt im allgemeinen 5-50 Gew.-%, vorzugsweise 15-25 Gew.-%.

Durch Extraktion der Perfluorether-Rohprodukte mit einer wäßrigen Lösung basischer Alkalimetallsalze und einfache Phasentrennung des Extrakts wird eine konzentrierte salzhaltige wäßrige Phase erhalten, neben einer schwereren Perfluoretherphase und einer leichteren wäßrigen Phase. Die genannte salzhaltige wäßrige Phase enthält neben den Salzen der perfluorierten Ethercarbonsäuren noch überschüssige basische Alkalimetallsalze sowie in emulgierter Form einen geringen Teil der Perfluorether (weniger als 1 Gew.%) , während sich deren Hauptmenge in der Perfluorether-Phase befindet. Wird die salzhaltige wäßrige Phase mit einer starken Säure, z.B. wäßriger Schwefelsäure, bei Raumtemperatur umgesetzt, so entstehen zwei Phasen. Die schwere Phase enthält die gewünschten Perfluorethercarbonsäuren sowie den erwähnten, in der salzhaltigen wäßrigen Phase emulgierten Anteil der perfluorierten Ether. Vorteilhaft wirkt sich der niedrige Gehalt an Wasser und an Fluoridionen aus, so daß die schwere Phase ohne weitere Reinigung zur elektrochemischen Herstellung von perfluorierten Ethern, wie in der JP-PS Sho 58 103 334 oder in der DE-OS 3 828 848 beschrieben, eingesetzt werden kann. Geeignete Säuren sind z.B. Schwefelsäure sowie Halogenwasserstoffsäuren, vorzugsweise Schwefelsäure, Salzsäure und Bromwasserstoffsäure, insbesondere 10-30 %ige Schwefelsäure.

Durch das erfindungsgemäße Verfahren wird die Ausbeute der Perfluorether-Synthese erhöht und die Menge an Abfallstoffen vermindert.

## Beispiele

Die im folgenden genannten Prozentzahlen sind stets Gewichtsprozente.

### Beispiel 1

6,1 kg Rohprodukt aus der elektrochemischen Synthese von $(C_3F_7\text{-}O\text{-}CF(CF_3)\text{-})_2$, das 0,4 % unumgesetzte Säure $C_3F_7\text{-}O\text{-}CF(CF_3)\text{-}COOH$ und 1,6 % Methylester $C_3F_7\text{-}O\text{-}CF(CF_3)\text{-}COOCH_3$ enthielt, wurden mit 366 g 20 %iger Kaliumhydroxidlösung in einer Glasrührapparatur durchmischt. Nach Phasentrennung wurden als mittlere Phase 242 g alkalische Lösung folgender Zusammensetzung erhalten:

56 % $C_3F_7\text{-}O\text{-}CF(CF_3)\text{-}COOK$
23 % $(C_3F_7\text{-}O\text{-}CF(CF_3)\text{-})_2$
21 % wäßrige Kaliumhydroxidlösung

230 g dieser Lösung wurden in einem 1 l-Glaskolben mit Magnetrührer und Tropftrichter bei 20 °C im Wasserbad vorgelegt und unter starkem Rühren mit 276 g 20 %iger Schwefelsäure während 20 min. versetzt. Nach vollständiger Zugabe wurden die entstandenen Phasen getrennt. Die schwere Phase (167 g) enthielt

65 % $C_3F_7\text{-}O\text{-}CF(CF_3)\text{-}COOH$,
32 % $(C_3F_7\text{-}O\text{-}CF(CF_3)\text{-})_2$,
3 % Wasser und
14 mg/l Fluorid.

Die leichte Phase (330 g) enthielt wäßrige Schwefelsäure und 170 mg/l Fluorid.

### Beispiel 2

16,6 kg eines durch Kolbe-Elektrolyse hergestellten Perfluorether-Rohproduktes, das

94 % $(C_3F_7\text{-}O\text{-}CF(CF_3)\text{-}CF_2\text{-}O\text{-}CF(CF_3)\text{-})_2$,
4,1 % $C_3F_7\text{-}O\text{-}CF(CF_3)\text{-}CF_2\text{-}O\text{-}CF(CF_3)\text{-}COOCH_3$ und
1,9 % $C_3F_7\text{-}O\text{-}CF(CF_3)\text{-}CF_2\text{-}O\text{-}CF(CF_3)\text{-}COOH$ enthielt,

wurden mit 1,6 kg einer 18 %igen wäßrigen Kaliumhydroxidlösung extrahiert. Durch Phasentrennung wurden 1584 g Salzlösung folgender Zusammensetzung als mittlere Phase erhalten:

68 % $C_3F_7\text{-}O\text{-}CF(CF_3)\text{-}CF_2\text{-}O\text{-}CF(CF_3)\text{-}COOK$
21 % $(C_3F_7\text{-}O\text{-}CF(CF_3)\text{-}CF_2\text{-}O\text{-}CF(CF_3)\text{-})_2$
11 % wäßrige Kaliumhydroxidlösung.

Nach Reaktion mit 1100 g 25 %iger wäßriger Schwefelsäure wurden 1340 g Perfluorethercarbonsäure-Lösung folgender Zusammensetzung als schwere Phase abgetrennt:
73,1 % $C_3F_7$-O-CF($CF_3$)-$CF_2$-O-CF($CF_3$)-COOH
24,7 % ($C_3F_7$-O-CF($CF_3$)-$CF_2$-O-CF($CF_3$)-)$_2$
2,2 % Wasser
5,0 mg/l Fluorid.

Die leichte Phase (1330 g) enthielt wäßrige Schwefelsäure und 100 mg/l Fluorid.

**Patentansprüche**

**1.** Verfahren zur Rückgewinnung von Perfluorethercarbonsäuren der allgemeinen Formel I

(I) $R_f$-COOH, worin

$R_f$ = $C_aF_{2a+1}$O-($C_3F_6$O)$_m$-CF($CF_3$)-
mit a = 1,2 oder 3
und m = 0,1,2 oder 3 ist

aus dem Rohprodukt der elektrochemischen Herstellung perfluorierter Ether, dadurch gekennzeichnet, daß man die in dem Rohprodukt enthaltene Perfluorethercarbonsäure der allgemeinen Formel I und deren Ester der allgemeinen Formel II

(II) $R_f$-COOR, worin

$R_f$ die unter (I) angegebene Bedeutung hat und
R = $C_cH_{2c+1}$ mit c = 1,2,3,4 oder 5
oder
R = -$C_dH_{2d+1}O_e$ mit d = 2,3,4,5 oder 6
und e = 1,2 oder 3 ist

mit Hilfe eines festen basischen Alkalimetallsalzes oder dessen Lösung abtrennt und durch Reaktion der dabei entstandenen Salze mit einer starken Säure in die entsprechende Perfluorethercarbonsäure der allgemeinen Formel I überführt.

**2.** Verfahren zur Rückgewinnung der beiden Perfluorethercarbonsäuren der allgemeinen Formel I und I'

(I) $R_f$-COOH, worin

$R_f$ = $C_aF_{2a+1}$O-($C_3F_6$O)$_m$-CF($CF_3$)-
mit a = 1,2 oder 3 und m = 0,1,2 oder 3 ist

(I,) $R_f$'-COOH, worin

$R_f$' = $C_bF_{2b+1}$O-($C_3F_6$O)$_n$-CF($CF_3$)-
mit b = 1,2 oder 3 und n = 0,1,2 oder 3 ist

aus dem Rohprodukt der elektrochemischen Umsetzung dieser Säuren oder ihrer löslichen Salze zu perfluorierten Ethern, dadurch gekennzeichnet, daß man die in dem Rohprodukt enthaltenen Perfluorethercarbonsäuren der allgemeinen Formeln I und I' und deren Ester der allgemeinen Formeln II and II'

(II) Rf-COOR

(II') $R_f$'-COOR

worin $R_f$ und $R_f$' die unter (I) und (I') angegebene Bedeutung haben
und R = $C_cH_{2c+1}$ mit c = 1,2,3,4 oder 5
oder R = $C_dH_{2d+1}O_e$ mit d = 2,3,4,5 oder 6
und e = 1,2 oder 3 ist

mit Hilfe eines festen basischen Alkalimetallsalzes oder dessen Lösung abtrennt und durch Reaktion der dabei entstandenen Salze mit einer starken Säure in die entsprechenden Perfluorethercarbonsäuren der allgemeinen Formeln I und I' überführt.

**3.** Verfahren zur Rückgewinnung einer Perfluorethercarbonsäure der allgemeinen Formel I

(I) $R_f$-COOH, worin

$R_f = C_aF_{2a+1}O-(C_3F_6O)_m-CF(CF_3)-$
mit a = 1,2 oder 3
und m = 0,1,2 oder 3 ist

aus dem Rohprodukt der elektrochemischen Umsetzung dieser Säure oder eines ihrer löslichen Salze zu einem perfluorierten Ether, dadurch gekennzeichnet, daß man die in dem Rohprodukt enthaltene Perfluorethercarbonsäure der allgemeinen Formel I und deren Ester der allgemeinen Formel II

(II) $R_f$-COOR, worin

$R_f$ die unter (I) angegebene Bedeutung hat und
$R = -C_cH_{2c+1}$ mit c = 1,2,3,4 oder 5
oder
$R = -C_dH_{2d+1}O_e$ mit d = 2,3,4,5 oder 6
und e = 1,2 oder 3 ist

mit Hilfe eines festen basischen Alkalimetallsalzes oder dessen Lösung abtrennt und durch Reaktion der dabei entstandenen Salze mit einer starken Säure in die entsprechende Perfluorethercarbonsäure der allgemeinen Formel I überführt.

**4.** Verfahren nach einem der Ansprüche 1 bis 3, dadurch gekennzeichnet, daß man als Alkalimetallsalz das Hydroxid oder Carbonat des Natriums oder Kaliums einsetzt.